# EUROPEAN PATENT APPLICATION

(11) **EP 4 397 315 A1**
(43) Date of publication of application: **10.07.2024**
(21) Application number: 22864474.6
(22) Date of filing: 29.08.2022
(51) Int. Cl.: A61K 39/39, A61K 9/133, A61K 9/14, A61K 47/34, A61K 47/36, A61P 35/00, A61P 37/04

(54) **COMPOSITION FOR ENHANCING IMMUNOGENICITY**

(30) Priority: 30.08.2021 JP 2021139683
(71) Applicant: Toray Industries, Inc., Tokyo 103-8666 (JP)
(72) Inventor: SHIBAHARA, Kyoko, Kamakura-shi, Kanagawa 248-8555 (JP); OKANO, Fumiyoshi, Kamakura-shi, Kanagawa 248-8555 (JP); PARK, Joonsik, Kamakura-shi, Kanagawa 248-8555 (JP)
(74) Representative: Mewburn Ellis LLP
(86) International application number: PCT/JP2022/032351
(87) International publication number: WO 2023/032891

(57) **Abstract**

The present invention relates to a composition for enhancing immunogenicity, containing, as an active ingredient, a particle comprising a modified amphiphilic polymer being an amphiphilic polymer in which a hydrophobic segment is poly(hydroxy acid) and a hydrophilic segment is polysaccharide and to which an immuno-stimulating factor is bound, and an antigen.

## Description

### Technical Field

The present invention relates to a composition for enhancing immunogenicity, containing, as an active ingredient, a particle comprising a modified amphiphilic polymer and an antigen, wherein the modified amphiphilic polymer is an amphiphilic polymer to which an immuno-stimulating factor is bound.

### Background Art

Since administration of antigens alone is insufficient for inducing immune responses to antigens sufficiently, adjuvants (immuno-stimulating factors) are used in combination. However, substances reported to have a high adjuvant effect are not approved for use in humans due to safety issues. While limited adjuvants including aluminum hydroxide and MF59 are used for medicaments, there is a demand for development of adjuvants not only ensuring safety, but also capable of inducing immune responses more potently.

In order to solve the above problems, microparticle adjuvants are progressively under development. Formation of particles allows for enclosure of antigens and immuno-stimulating factors, and thus an enhancement in safety and an enhancement in delivery efficiency to target cells are expected. There have been proposed microparticle adjuvants with diverse materials such as fatty acids, biodegradable polymers, and viral proteins (Non Patent Literature 1 and 2).

In recent years, there has been a reported technique relating to an antigen-adjuvant microparticle complex, wherein an antigen is enclosed in an adjuvant microparticle consisting of an amphiphilic polymer in which a hydrophobic segment is poly(hydroxy acid) and a hydrophilic segment is polysaccharide including β-glucan (Patent Literature 1 and 2). This technique has successfully induced a high immune response to an antigen by a small amount of the antigen and a small number of administrations. However, to date, an effective adjuvant having much better performances than conventional adjuvants has not been realized by techniques using existing microparticles, although development thereof has been hoped.

### Citation List

### Patent Literature

Patent Literature 1: WO2010/098432
Patent Literature 2: WO2015/053354

### Non Patent Literature

Non Patent Literature 1: Immunology, vol. 117, 2006, pages 77 to 88
Non Patent Literature 2: Nature Materials, vol. 10, 2011, pages 243 to 251

### Summary of Invention

### Technical Problem

An object of the present invention is to provide a composition for enhancing immunogenicity having potent immuno-stimulating capacity, by enhancing immuno-stimulating action of a particle composed of an amphiphilic polymer in which a hydrophobic segment is poly(hydroxy acid) and a hydrophilic segment is polysaccharide.

### Solution to Problem

In order to overcome the above problems, the present inventor has found that a composition for enhancing immunogenicity containing a particle comprising a modified amphiphilic polymer and an antigen wherein the modified amphiphilic polymer is an amphiphilic polymer to which an immuno-stimulating factor is bound, is effective, and thus has completed the present invention.

In other words, the present invention has the constitution as shown in the following (1) to (19).
(1) A composition for enhancing immunogenicity, containing, as an active ingredient, a particle comprising a modified amphiphilic polymer and an antigen, wherein the modified amphiphilic polymer is an amphiphilic polymer in which a hydrophobic segment is poly(hydroxy acid) and a hydrophilic segment is polysaccharide and to which an immuno-stimulating factor is bound.
(2) The composition for enhancing immunogenicity according to (1), wherein the composition does not comprise any lipid as a constituent of the particle other than the antigen.
(3) The composition for enhancing immunogenicity according to (1) or (2), wherein the particle further comprises a non-modified amphiphilic polymer, wherein the non-modified amphiphilic polymer is an amphiphilic polymer in which a hydrophobic segment is poly(hydroxy acid) and a hydrophilic segment is polysaccharide and to which no immuno-stimulating factor is bound.
(4) The composition for enhancing immunogenicity according to any of (1) to (3), wherein the polysaccharide is dextran, β-glucan, mannan, chitin, chitosan, gellan gum, alginic acid, hyaluronic acid or pullulan.
(5) The composition for enhancing immunogenicity according to (4), wherein the β-glucan is a polymer of glucose linked by one or more β-1,3 bonds and/or one or more β-1,6 bonds.
(6) The composition for enhancing immunogenicity according to (4) or (5), wherein the β-glucan is black yeast glucan, curdlan, pachyman, laminaran, lichenan, schizophyllan, lentinan, scleroglucan or pachymaran.
(7) The composition for enhancing immunogenicity according to any of (1) to (6), wherein the poly(hydroxy acid) is poly(lactic-co-glycolic acid), polylactic acid or polyglycolic acid.
(8) The composition for enhancing immunogenicity according to any of (1) to (7), wherein binding between the amphiphilic polymer and the immuno-stimulating factor in the modified amphiphilic polymer is covalent binding.
(9) The composition for enhancing immunogenicity according to any of (1) to (8), wherein a portion to which the immuno-stimulating factor is bound in the modified amphiphilic polymer is the hydrophilic segment.
(10) The composition for enhancing immunogenicity according to any of (1) to (9), wherein the immuno-stimulating factor is a ligand or agonist binding to a Toll-like receptor (TLR), a NOD-like receptor (NLR), a RIG-like receptor or a C-type lectin receptor (CLR), or a stimulator of interferon gene (STING).
(11) The composition for enhancing immunogenicity according to (10), wherein the ligand or agonist binding to a Toll-like receptor (TLR) is a ligand or agonist binding to TLR2, TLR3, TLR4, TLR5, TLR7, TLR8, TLR9 or TLR11.
(12) The composition for enhancing immunogenicity according to (10) or (11), wherein the ligand or agonist binding to a Toll-like receptor (TLR) is any of the following (i) to (vii):
   (i) a ligand or agonist binding to TLR2 selected from the group consisting of peptidoglycan, lipoprotein, lipopolysaccharide, and zymosan;
   (ii) a ligand or agonist binding to TLR3 selected from the group consisting of poly(I:C) and poly(A:U);
   (iii) a ligand or agonist binding to TLR4 selected from the group consisting of lipopolysaccharide (LPS), HSP60, RS09, and MPLA;
   (iv) flagellin as a ligand or agonist binding to TLR5;
   (v) a ligand or agonist binding to TLR7 or 8 selected from the group consisting of an imidazoquinoline compound (imidazoquinoline species) and single-strand RNA;
   (vi) a ligand or agonist binding to TLR9 selected from the group consisting of bacterial DNA, unmethylated CpG DNA, hemozorin, ODN1585, ODN1668, and ODN1826; and
   (vii) a ligand or agonist binding to TLR11 selected from the group consisting of profilin and uropathogenic bacteria.
(13) The composition for enhancing immunogenicity according to any of (1) to (12), wherein the number of molecules of the immuno-stimulating factor binding to one molecule of the modified amphiphilic polymer is 1 to 100.
(14) The composition for enhancing immunogenicity according to any of (1) to (13), wherein an average particle size of the particle is 0.1 to 50 µm.
(15) A medicine containing the composition for enhancing immunogenicity according to any of (1) to (14), as an active ingredient.
(16) A vaccine containing the composition for enhancing immunogenicity according to any of (1) to (14), as an active ingredient.
(17) A vaccine for treatment and/or prevention of cancer, containing the composition for enhancing immunogenicity according to any of (1) to (14), as an active ingredient.
(18) A method for enhancing immunogenicity, comprising administering the composition for enhancing immunogenicity according to any of (1) to (14), to a subject.
(19) A method for treating and/or preventing cancer, comprising administering the composition for enhancing immunogenicity according to any of (1) to (14), the medicine according to (15), or the vaccine according to (16) or (17), to a subject.

The present specification encompasses the disclosure of Japanese Patent Application No. 2021-139683 which is the basis of the priority claim of the present application.

### Advantageous Effects of Invention

The present invention provides a composition for enhancing immunogenicity, which can allow for immuno-stimulating more potently than conventional ones.

### Brief Description of Drawing

[Figure 1] Figure 1 represents the results of GPC measurement of dextran-NH₂.
[Figure 2] Figure 2 represents the results of GPC measurement of dextran-PLGA.
[Figure 3] Figure 3 represents the results of ¹H-NMR measurement of R848-dextran-PLGA.
[Figure 4] Figure 4 represents the results of GPC measurement of black yeast glucan-NH₂.
[Figure 5] Figure 5 represents the results of GPC measurement of black yeast glucan-PLGA.
[Figure 6] Figure 6 represents the results of ¹H-NMR measurement of R848-black yeast glucan-PLGA.
[Figure 7] Figure 7 represents the results of DLS measurement of an OVA-containing R848-modified dextran-PLGA particle.
[Figure 8] Figure 8 represents the results of DLS measurement of an OVA-containing R848-modified black yeast glucan-PLGA particle.
[Figure 9] Figure 9 represents the results of DLS measurement of a TRP2-containing R848-modified black yeast glucan-PLGA particle.
[Figure 10] Figure 10 represents the results of activation of mouse bone marrow-derived dendritic cells by a composition for enhancing immunogenicity.

### Description of Embodiments

The present invention relates to an immunogenic composition (in particular, composition for enhancing immunogenicity) containing, as an active ingredient, a particle comprising a modified amphiphilic polymer and an antigen, wherein the modified amphiphilic polymer is an amphiphilic polymer in which a hydrophobic segment is poly(hydroxy acid) and a hydrophilic segment is polysaccharide and to which an immuno-stimulating factor is bound. The present invention preferably relates to a particle comprising the modified amphiphilic polymer, an antigen, and a non-modified amphiphilic polymer, wherein the non-modified amphiphilic polymer is an amphiphilic polymer in which a hydrophobic segment is poly(hydroxy acid) and a hydrophilic segment is polysaccharide and to which no immuno-stimulating factor is bound.

The amphiphilic polymer constituting a particle together with an antigen is described. The term "amphiphilic" means having both properties of hydrophilicity and hydrophobicity. When the solubility in water of a certain portion (segment) is higher than that of other portions, the certain portion (segment) is referred to as being hydrophilic. The hydrophilic portion is desirably soluble in water, but may be hardly soluble in water as long as it has higher solubility in water as compared with other portions. When the solubility in water of a certain portion (segment) is lower than that of other portions, the certain portion (segment) is referred to as being hydrophobic. The hydrophobic portion is desirably insoluble in water, but may be soluble as long as it has lower solubility in water as compared with other portions.

The term "amphiphilic polymer" means a polymer having the above amphiphilicity as the whole molecule. The amphiphilic "polymer" indicates that a hydrophilic segment or a hydrophobic segment, or both thereof in an amphiphilic molecule has a molecular structure constituted from a repeated structure of a minimum unit (monomer). The structure of the amphiphilic polymer in the present invention is not particularly limited, and specific examples thereof include: a straight-chained block-type polymer in which polysaccharide and poly(hydroxy acid) are connected; a branched polymer having branches, comprising a plurality of polysaccharides or poly(hydroxy acids); a graft-type polymer comprising a main chain of polysaccharide and a side chain of poly(hydroxy acid); and a graft-type polymer comprising a main chain of poly(hydroxy acid) and a side chain of polysaccharide. The structure of the amphiphilic polymer in the present invention is preferably a straight-chained block-type polymer in which polysaccharide and poly(hydroxy acid) are connected.

In the present invention, the hydrophilic segment of the amphiphilic polymer is polysaccharide. The hydrophilic segment is not particularly limited as long as it is polysaccharide, and specific examples thereof include dextran, β-glucan, mannan, chitin, chitosan, gellan gum, alginic acid, hyaluronic acid or pullulan. The hydrophilic segment is preferably dextran or β-glucan.

The glucan refers to glucose-containing polysaccharides, and the β-glucan refers to glucan containing one or more β-bonds between glucose subunits. In other words, the β-glucan for use in the present invention contains a β-bond, and may be one containing only a β-bond. The β-glucan for use in the present invention may be branched or linear. Examples of preferable β-glucan include β-glucan containing one or more β-1,3 bonds and/or one or more β-1,6 bonds, or β-glucan containing one or more β-1,2 bonds and/or a β-1,4 bond, and β-glucan containing one or more β-1,3 bonds and/or one or more β-1,6 bonds is more preferable, and β-glucan containing one or more β-1,3 bonds is further preferable. Specific examples of the β-glucan containing one or more β-1,3 bonds include curdlan, pachyman, laminaran, lichenan, schizophyllan, lentinan, scleroglucan, black yeast glucan (preferably, black yeast-derived β-1,3 glucan or β-1,6 glucan) or pachymaran, preferably include curdlan, pachyman, laminaran, schizophyllan, scleroglucan, black yeast glucan or pachymaran.

Examples of linear β-glucan containing one or more β-1,3 bonds include β-glucan mainly containing a β-1,3 bond (for example, curdlan or pachyman), or β-glucan containing a β-1,3 bond and a β-bond other than a β-1,3 bond (for example, laminaran or lichenan).

Examples of branched β-glucan containing one or more β-1,3 bonds include β-glucan containing a β-1,3 bond and a β-1,6 bond (for example, schizophyllan, lentinan, scleroglucan, or black yeast glucan).

The β-glucan for use in the present invention may be derivatized β-glucan. Examples of such derivatization include addition reaction of a carboxymethyl group, or oxidative cleavage reaction. Examples of the derivatized β-glucan include carboxymethyl curdlan obtained by addition of a carboxymethyl group to curdlan, or pachymaran obtained by cleavage of pachyman.

The number-average molecular weight of polysaccharide is not particularly limited, and is preferably 500 to 100,000, more preferably 500 to 50,000, further preferably 1,000 to 10,000, for example, 1,000 to 8,000, 1,000 to 6,000, or 1,000 to 4,000. The number-average molecular weight is an average molecular weight calculated by a method in which weighting for the size of a molecule is not considered. The number-average molecular weight of polysaccharide can be determined by gel permeation chromatography (GPC).

In the present invention, the hydrophobic segment of the amphiphilic polymer is poly(hydroxy acid). The poly(hydroxy acid) is not particularly limited, and is preferably a biocompatible polymer without remarkable adverse effect upon administration into an organism. The biocompatibility herein refers to having an LD50 of 2,000 mg/kg or more when the polymer is orally administered to a rat. The poly(hydroxy acid) may be a copolymer of a plurality types of hydroxy acids, and is preferably a polymer of two or less types of hydroxy acids.

Preferable examples of the poly(hydroxy acid) include polyglycolic acid, polylactic acid, poly(2-hydroxybutyric acid), poly(2-hydroxyvaleric acid), poly(2-hydroxycaproic acid), poly(2-hydroxycapric acid), poly(malic acid), or any derivative and copolymer of such polymer compounds. The poly(hydroxy acid) is preferably poly(lactic-co-glycolic acid), polylactic acid or polyglycolic acid, and more preferably poly(lactic-co-glycolic acid). In a case where the poly(hydroxy acid) is poly(lactic-co-glycolic acid), the compositional ratio (lactic acid/glycolic acid) (mol/mol) of the poly(lactic-co-glycolic acid) is not particularly limited as long as the objects of the present invention are achieved, and is preferably 99/1 to 1/99, more preferably 80/20 to 20/80, for example, 60/40 to 40/60, or 50/50.

The number-average molecular weight of poly(hydroxy acid) is not particularly limited, and is preferably 500 to 1,000,000, more preferably 500 to 100,000, further preferably 500 to 50,000, for example, 500 to 40,000, 500 to 30,000, 500 to 20,000, or 500 to 15,000. The number-average molecular weight of poly(hydroxy acid) can be determined from the difference between the number-average molecular weight of the amphiphilic polymer in which a hydrophobic segment is poly(hydroxy acid) and a hydrophilic segment is polysaccharide, and the number-average molecular weight of polysaccharide.

The number-average molecular weight of the amphiphilic polymer constituting the particle (non-modified amphiphilic polymer to which no immuno-stimulating factor is bound) is not particularly limited, and is preferably 1,000 to 1,000,000, more preferably 1,000 to 100,000, further preferably 9,000 to 50,000, for example, 9,000 to 40,000, 9,000 to 30,000, 9,000 to 20,000, or 9,000 to 15,000. The number-average molecular weight of the amphiphilic polymer can be determined by gel permeation chromatography (GPC).

The amphiphilic polymer may be produced by a known method. Specific examples of the method include: a production method comprising adding poly(hydroxy acid) to polysaccharide and performing condensation reaction; or a production method comprising adding a hydroxy acid activated monomer to polysaccharide and performing polymerization reaction.

In a case where the amphiphilic polymer is a straight-chained block-type polymer in which polysaccharide and poly(hydroxy acid) are connected, the polymer may be produced by a known method. Specific examples of the method include a production method comprising performing condensation reaction of a poly(hydroxy acid) copolymer to a reducing terminal of polysaccharide of the amphiphilic polymer using an activator of a terminal functional group (Macromol. Rapid Commun., 31, p.1664-1684 (2010)).

In a case where the amphiphilic polymer is a graft-type polymer comprising a main chain of polysaccharide and a side chain of poly(hydroxy acid), the polymer can be produced according to the following method (1), (2), or (3).

(1) A method of producing a graft-type polymer, by adding a hydroxy acid activated monomer to polysaccharide and performing polymerization reaction in the presence of a tin catalyst to introduce a poly(hydroxy acid) to the polysaccharide (Macromolecules, 31, p. 1032-1039 (1998)).
(2) A method of producing a graft-type polymer, by activating some unprotected hydroxyl groups of polysaccharide in which most hydroxyl groups are protected with substituents, with a base, then adding a hydroxy acid activated monomer thereto to introduce a graft chain of poly(hydroxy acid), and finally removing protect groups (Polymer, 44, p. 3927-3933, (2003)).
(3) A method of producing a graft-type polymer, by condensation reaction of a copolymer of poly(hydroxy acid) to polysaccharide using a dehydrating agent and/or an activator of a functional group (Macromolecules, 33, p. 3680-3685 (2000)).

The term "modified amphiphilic polymer" refers to a polymer obtained by binding an immuno-stimulating factor to the amphiphilic polymer. The binding herein may be non-covalent binding or covalent binding, and is preferably covalent binding. The non-covalent binding is preferably hydrophobic interaction, and may be ion binding (electrostatic interaction), hydrogen binding, coordination linkage, van der Waals binding, or physical adsorption, or may be a combination thereof.

The term "immuno-stimulating factor" here means a substance which is a factor activating one or more immune cells and can maintain and/or enhance the immune function of such cells. The term "immune cells" herein include, for example, T lymphocytes, B lymphocytes, NK cells, monocytes, dendritic cells, granulocytes, macrophages, bone marrow-derived inhibitory cells, Langerhance cells and a group of precursor cells thereof, and a group of the immune cells in tumors.

The immuno-stimulating factor for use in the present invention is not particularly limited, and specific examples thereof include a ligand or agonist binding to a Toll-like receptor (TLR), a NOD-like receptor (NLR), a RIG-like receptor, a C-type lectin receptor (CLR), or a stimulator of interferon gene (STING). The immuno-stimulating factor is preferably a ligand or agonist binding to TLR.

Specific examples of TLR include TLR2, TLR3, TLR4, TLR5, TLR7, TLR8, TLR9 or TLR11, and specific examples of the ligand or agonist binding thereto include the following (i) to (vii).

(i) A ligand or agonist binding to TLR2 selected from the group consisting of peptidoglycan, lipoprotein, lipopolysaccharide, and zymosan.
(ii) A ligand or agonist binding to TLR3 selected from the group consisting of poly(I:C) and poly(A:U).
(iii) A ligand or agonist binding to TLR4 selected from the group consisting of lipopolysaccharide (LPS), HSP60, RS09, and MPLA.
(iv) Flagellin as a ligand or agonist binding to TLR5.
(v) A ligand or agonist binding to TLR7 or 8 selected from the group consisting of an imidazoquinoline compound and single-strand RNA.
(vi) A ligand or agonist binding to TLR9 selected from the group consisting of bacterial DNA, unmethylated CpG DNA, hemozorin, ODN1585, ODN1668, and ODN1826.
(vii) A ligand or agonist binding to TLR11 selected from the group consisting of profilin and uropathogenic bacteria.

The immuno-stimulating factor in the present invention is preferably a ligand or agonist binding to TLR7 or 8 (TLR7/8 ligand or agonist), more preferably the ligand or agonist of the above item (v).

Preferable specific examples of the imidazoquinoline compound in the above item (v) include any compound described in US Patent No. 8,951,528 and any compound described in WO2015/103989, and examples thereof include 4-amino-2-(ethoxymethyl)-a,a-dimethyl-1H-imidazo[4,5-c]quinoline-1-ethanol (Resiquimod (R848)), 1-(2-methylpropyl)-1H-imidazo[4,5-c]quinoline-4-amine (Imiquimod), 1-(4-amino-2-ethylaminomethylimidazo-[4,5-c]quinolin-1-yl)-2-methylpropan-2-ol (Gardiquimod), N-[4-(4-amino-2-ethyl-1H-imidazo[4,5-c]quinolin-1-yl)butyl-]methanesulfonamide (PF-4878691), 4-amino-aa-dimethyl-2-methoxyethyl-1H-imidazo[4,5-c]quinoline-1-ethanol, 1-(2-(3-(benzyloxy)propoxy)ethyl)-2-(ethoxymethyl)-1H-imidazo[4,5-c]quinolin-4-amine, 4-amino-2-ethoxymethyl-aa-dimethyl-6,7,8,9-tetrahydro-1H-imidazo[4,5-c]quinoline-1-ethanol, N-(2-{2-[4-amino-2-(2-methoxyethyl)-1H-imidazo[4,5-c]quinolin-1-yl]eyhoxy}ethyl)-n'-phenylurea, 1-2-amino-2-methylpropyl)-2-(ethoxymethyl)-1H-imidazo[4,5-c]quinolin-4-amine, 1-{4-[(3,5-dichlorophenyl)sulfonyl]butyl}-2-ethyl-1H-imidazo[4,5-c]quinolin-4-amine, N-(2-{2-[4-amino-2-(ethoxymethyl)-1H-imidazo[4,5-c] quinolin-1-yl] ethoxy} ethyl)-n' -cyclohexylurea, N-{ 3 - [4-amino-2-(ethoxymethyl)-1H-imidazo[4,5-c]quinolin-1-yl]propyl}-n'-(3-cyanophenyl)thiourea, N-[3-(4-amino-2-butyl-1H-imidazo[4,5-c]quinolin-l-yl)-2,2-dimethylpropyl]benzamide, 2-butyl-1-[3-(methylsulfonyl)propyl]-1H-imidazo[4,5-c]quinolin-4-amine, and derivatives thereof, but the imidazoquinoline compound is not particularly limited as long as it can bind to TLR7 or TLR8.

Specific examples of the ligand or agonist binding to a NOD-like receptor (NLR) include M-TriDAP and PGN, as well as the ligand or agonist binding to NOD1 such as Tri-DAP, iE-DAP, and C12-iE, and the ligand or agonist binding to NOD2 such as MDP, N-glycosyl-MDP, Murabutide, M-TriLyS-D-ASN, M-TriLYS, and L18-MDP.

Specific examples of the ligand or agonist binding to a RIG-like receptor include 5'ppp-dsRNA, poly(dA:dT), poly(dG:dC), and poly(I:C).

Specific examples of the ligand or agonist binding to a C-type lectin receptor (CLR) include trehalose 6,6-dibehenate, zymosan, WGP, HKSC, HKCA, and curdlan AL.

Specific examples of the ligand or agonist binding to a stimulator of interferon gene (STING) include c-di-GMP, c-di-AMP, 2'3'-cGAMP, 3'3'-cGAMP or 2'2'-cGAMP.

The portion to which the immuno-stimulating factor is bound in the modified amphiphilic polymer is not particularly limited, and is preferably a polysaccharide segment. The ratio (molar ratio) of the immuno-stimulating factor to the amphiphilic polymer (the number of molecules of the immuno-stimulating factor binding to one molecule of the modified amphiphilic polymer) is not particularly limited as long as the objects of the present invention are achieved, and is preferably 1 to 100, more preferably 1 to 50, further preferably 1 to 30, particularly preferably 1 to 10, with respect to the modified amphiphilic polymer.

The number-average molecular weight of the modified amphiphilic polymer is not particularly limited, and is preferably 1,000 to 1,000,000, more preferably 1,000 to 100,000, further preferably 2,000 to 50,000, for example, 2,000 to 40,000, 2,000 to 30,000, 2,000 to 20,000, or 2,000 to 15,000. The number-average molecular weight of the modified amphiphilic polymer can be determined by gel permeation chromatography (GPC).

The modified amphiphilic polymer may be produced by a known method, and can be specifically produced according to the following method (1) or (2).

### (1) Method for binding immuno-stimulating factor to polysaccharide segment of amphiphilic polymer

Examples of the above method include the following methods:
a production method, which comprises quantitatively activating a hydroxyl group of polysaccharide with a reaction reagent for activating a hydroxyl group of a polysaccharide main chain, for example, 1,1'-carbonyldiimidazole (CDI) or disuccinimidyl carbonate (DSC) without particular limitations, and then performing condensation reaction of the above activated hydroxyl group and a reactive functional group to be covalently bound to the activated hydroxyl group separately introduced to an immuno-stimulating factor, for example, an amino group;
a production method which comprises converting a hydroxyl group of polysaccharide into other more reactive functional group, for example, an amino group, a carboxyl group, a maleimide group, or a thiol group without particular limitations, and then performing condensation reaction of the polysaccharide and an immuno-stimulating factor to which a functional group to be bound to the reactive functional group is separately introduced; or
a production method which comprises converting a hydroxyl group of polysaccharide into a cationic amino group or an anionic carboxyl group, and then ionically binding the amino group or carboxyl group to a carboxyl group or amino group having opposite charge separately introduced into an immuno-stimulating factor through electrostatic interaction.

### (2) Method for binding immuno-stimulating factor to poly(hydroxy acid) segment of amphiphilic polymer

The α-terminal of poly(hydroxy acid) can be connected with polysaccharide to form a copolymer, and various reactive functional groups, for example, an amino group, a carboxyl group, a maleimide group, or a thiol group, without particular limitation, can be introduced to the ω-terminal of poly(hydroxy acid). Examples of the above method include the following methods:
a production method which comprises performing condensation reaction of the above poly(hydroxy acid) and an immuno-stimulating factor to which a functional group to be bound to the reactive functional group introduced to the ω-terminal of poly(hydroxy acid) is separately introduced; or
a production method which comprises converting the ω-terminal of poly(hydroxy acid) into a cationic amino group or anionic carboxyl group, and then ionically binding the amino group or carboxyl group to a carboxyl group or amino group having opposite charge separately introduced into an immuno-stimulating factor through electrostatic interaction.

The modified amphiphilic polymer is preferably water-insoluble as a whole so as not to be rapidly excreted *in vivo,* in order to keep immuno-stimulating action for a long period. The term "water-insoluble" herein refers to having a solubility in water of 1 g (amphiphilic polymer)/100 ml (water) or less.

The particle being an active ingredient of the composition for enhancing immunogenicity of the present invention comprises an antigen and the modified amphiphilic polymer as constituents, and preferably further comprises a non-modified amphiphilic polymer to which no immuno-stimulating factor is bound. The particle in the present invention can have remarkably enhanced immuno-stimulating capacity as compared with: a particle comprising an antigen and the non-modified amphiphilic polymer and not comprising any modified amphiphilic polymer; a mixture of the above particle and an immuno-stimulating factor; or an antigen alone.

In a case where constituents of the particle other than the antigen comprise a combination of the modified amphiphilic polymer and the non-modified amphiphilic polymer, the combination is not particularly limited as long as the particle has immuno-stimulating capacity, and the hydrophilic segment may be a different polymer or the same type of polymer between the modified amphiphilic polymer and the non-modified amphiphilic polymer, and similarly, the hydrophobic segment may also be a different polymer or the same type of polymer between the modified amphiphilic polymer and the non-modified amphiphilic polymer. In a case where each segment is a different polymer between the modified amphiphilic polymer and the non-modified amphiphilic polymer, three or more types of polymers may be adopted. The linkage mode of the hydrophilic segment and the hydrophobic segment may be different or the same between the modified amphiphilic polymer and the non-modified amphiphilic polymer. For example, a combination may be adopted in which the modified amphiphilic polymer is a block-type polymer and the non-modified amphiphilic polymer is a graft-type polymer. Each segment is preferably formed from the same type of polymer between the modified amphiphilic polymer and the non-modified amphiphilic polymer.

In a case where constituents of the particle other than the antigen comprise a combination of the modified amphiphilic polymer and the non-modified amphiphilic polymer, the ratio of the weight of the modified amphiphilic polymer with respect to the total weight of the modified amphiphilic polymer and the non-modified amphiphilic polymer is preferably 0.01 to 99.99% by weight, more preferably 0.1 to 99.9% by weight, further preferably 1 to 99% by weight, still further preferably 5 to 95% by weight, particularly preferably 5 to 80% by weight.

Constituents of the particle other than the antigen may comprise a component other than the amphiphilic polymer. Specific examples thereof can include lipid well-known as a constituent of a liposome or lipid nanoparticle that is a particle capable of enclosing the antigen. However, preferably, constituents of the particle other than the antigen comprise no lipid, since expected effects are sufficiently obtained in the present invention even without any lipid, as is evident from Examples.

The structure of the particle is not particularly limited. However, it is preferable that the particle has a structure in which the hydrophilic segment of the amphiphilic polymer constituting the particle is located inside the particle and the hydrophobic segment serves as an outer layer of the particle in order to form a complex of the particle and an antigen, because, in such case, the antigen is enclosed in the particle and can be more stably retained.

The method for producing the particle is not particularly limited, and examples thereof include a drying in liquid method, a spray-drying method, and a pulverizing method. The particle in the present invention is preferably produced by a drying in liquid method.

Examples of the method for producing the particle by a drying in liquid method include an O/W emulsion method, a W/O/W emulsion method, and a S/O/W emulsion method.

The particle, when produced by an O/W emulsion method, can be produced by, for example, a step of mixing a water-immiscible organic solvent dissolving a powder of the amphiphilic polymer constituting the particle, and an aqueous solution dissolving a surface modifier and an antigen, to prepare an O/W emulsion solution, and a step of removing the water-immiscible organic solvent from the O/W emulsion solution to obtain the particle.

The particle, when produced by a W/O/W emulsion method, can be produced by, for example, a step of mixing an aqueous solvent dissolving an antigen and a water-immiscible organic solvent dissolving a powder of the amphiphilic polymer constituting the particle, to prepare a W/O emulsion solution, a step of mixing the W/O emulsion solution and an aqueous surface modifier solution to prepare a W/O/W emulsion solution, and a step of removing the water-immiscible organic solvent from the W/O/W emulsion solution to obtain the particle.

The particle, when produced by a S/O/W emulsion method, can be produced by, for example, a step of mixing an aqueous solvent dissolving an antigen and a water-immiscible organic solvent dissolving a powder of the amphiphilic polymer constituting the particle, to prepare a W/O emulsion solution, a step of removing the solvent from the W/O emulsion solution to obtain a solid content, a step of dispersing the solid content in the water-immiscible organic solvent to obtain a S/O suspension solution, a step of mixing the S/O suspension solution and an aqueous surface modifier solution to prepare a S/O/W emulsion solution, and a step of removing the water-immiscible organic solvent from the S/O/W emulsion solution to obtain the particle.

The content ratio of the antigen in the particle (antigen/particle) is preferably 0.01 to 20% by weight, more preferably 0.1 to 10% by weight, for example, 0.5 to 10% by weight. Examples of the method for quantitatively determining the content ratio of the antigen include a method comprising extracting the antigen from the particle using an organic solvent, and quantitatively determining the content ratio of the antigen by gel electrophoresis or liquid chromatography.

The surface modifier used for particle preparation is preferably a water-soluble polymer or a surfactant. The water-soluble polymer herein is a polymer compound having a solubility in water of 1 g (water-soluble polymer)/100 ml (water) or more.

Examples of the water-soluble polymer serving as the surface modifier include polyethylene glycol, polyvinylpyrrolidone, polyvinyl alcohol, polyethylenimine, polyacrylic acid, polymethacrylic acid, poly-1,3-dioxolane, 2-methacryloyloxyethyl phosphorylcholine polymer, poly-1,3,6-trioxane, polyamino acid, peptide, protein, sugar (monosaccharides, oligosaccharides, and polysaccharides), and polyvinyl alcohol is more preferable.

Examples of the surfactant serving as the surface modifier include a nonion activator such as polyoxyethylene polyoxypropylene glycol, sucrose fatty acid ester, polyoxyethylene fatty acid ester, polyoxyethylene sorbitan monofatty acid ester, polyoxyethylene sorbitan difatty acid ester, polyoxyethylene glycerin monofatty acid ester, polyoxyethylene glycerin difatty acid ester, polyglyceryl fatty acid ester, polyoxyethylene castor oil, or polyoxyethylene hydrogenated castor oil, alkyl sulfate such as sodium lauryl sulfate, ammonium lauryl sulfate, or sodium stearyl sulfate, or lecithin, and polyoxyethylene polyoxypropylene glycol is more preferable.

The water-immiscible organic solvent used in particle preparation preferably can dissolve the amphiphilic polymer and the modified amphiphilic polymer, and hardly dissolves or does not dissolve polysaccharide. The solubility in water of the water-immiscible organic solvent is preferably 30 g (water-immiscible organic solvent)/100 ml (water) or less. Specific examples of the water-immiscible organic solvent include ethyl acetate, isopropyl acetate, butyl acetate, dimethyl carbonate, diethyl carbonate, methylene chloride, and chloroform.

The aqueous solvent used in particle preparation is an aqueous solution containing water and optionally a water-soluble component. Examples of the water-soluble component include inorganic salts, sugar, organic bases, amino acid, peptide, protein, and nucleic acid.

The surface modifier used in the above production process may be bound to the particle surface. The binding herein may be non-covalent binding or covalent binding. The non-covalent binding is preferably hydrophobic interaction, and may be ion binding (electrostatic interaction), hydrogen binding, coordination linkage, van der Waals binding, or physical adsorption, or may be combination thereof.

The average particle size of the particle is preferably 0.1 to 50 µm, more preferably 0.1 to 25 µm, further preferably 0.1 to 10 µm, particularly preferably 0.1 to 1 µm, for example, 0.2 to 1 µm, or 0.3 to 1 µm. The average particle size herein can be determined with a dynamic light scattering apparatus (DLS: for example, ELS-Z, OTSUKA ELECTRONICS CO., LTD) according to a cumulant method.

The term "composition for enhancing immunogenicity" in the present invention refers to a composition that can enhance an immune response to an antigen *in vivo* containing, as an active ingredient, the particle comprising the modified amphiphilic polymer and an antigen, wherein the modified amphiphilic polymer is an amphiphilic polymer to which an immuno-stimulating factor is bound. The type of the immune response generated by the composition for enhancing immunogenicity is not limited. The type of the immune response generated includes a Th1-type immune response or a Th2-type immune response. Though it is known that one of such immune responses is preferentially generated depending on the types of an antigen, an immuno-stimulating factor, an administration site, and an administration method, the present invention can generate both Th1-type and Th2-type immune responses.

The term "antigen" in the present invention refers to a substance which triggers immunity *in vivo,* and thus can be utilized as a vaccine for treatment and/or prevention of disease. An immune response triggered by the antigen can be enhanced by using, as an active ingredient, the particle containing the modified amphiphilic polymer with the immuno-stimulating factor attached, and the antigen, of the present invention.

Examples of the antigen include peptide, protein, glycoprotein, glycolipid, lipid, carbohydrate, nucleic acid, and polysaccharide, as well as virus, fungus body, allergen, tissue and cell comprising any of them. Specific examples include a pollen-derived antigen, a hepatitis A virus-derived antigen, a hepatitis B virus-derived antigen, a hepatitis C virus-derived antigen, a hepatitis D virus-derived antigen, a hepatitis E virus-derived antigen, a hepatitis F virus-derived antigen, an HIV virus-derived antigen, an influenza virus-derived antigen, a herpesvirus (HSV-1, HSV-2)-derived antigen, an anthrax-derived antigen, a chlamydia-derived antigen, a pneumococcus-derived antigen, a Japanese encephalitis virus-derived antigen, a measles virus-derived antigen, a rubella virus-derived antigen, a Clostridium tetani-derived antigen, a varicella virus-derived antigen, a SARS virus-derived antigen, an EB virus-derived antigen, a papillomavirus-derived antigen, a Helicobacter pylori-derived antigen, a rabies virus-derived antigen, a West Nile virus-derived antigen, a hantavirus-derived antigen, a streptococcus-derived antigen, a staphylococcus-derived antigen, a Bordetella pertussis-derived antigen, a Mycobacterium tuberculosis-derived antigen, a Plasmodium-derived antigen, a poliomyelitis virus-derived antigen, various zoonotic infection-derived antigens, various food allergy-derived antigens, and a self-antigen.

Other preferable examples of the antigen include a cancer antigen. The cancer antigen is a substance derived from a protein specifically expressed in cancer cells, and is an antigen which is to be administered from the outside of a living boy to the inside of the living body to exert an effect of treatment and/or prevention of cancer by an immune response.

The composition for enhancing immunogenicity of the present invention can be used for treatment and/or prevention of disease such as cancer, infection, or allergy. Examples of the cancer for which the composition for enhancing immunogenicity of the present invention can be used include basal cell cancer, Paget's disease, brain tumor, urinary bladder cancer, esophageal cancer, leukemia, lymphoma, liver cancer, gallbladder cancer, sarcoma, mastocytoma, adrenal cortex cancer, Ewing's tumor, Hodgkin's lymphoma, mesothelioma, multiple myeloma, thyroid cancer, skin cancer (for example, melanoma), lung cancer, pharynx cancer, gastric cancer, pancreatic cancer, colon cancer, kidney cancer, urinary bladder cancer, breast cancer, uterus cancer, ovarian cancer, prostate cancer, and head and neck cancer. Examples of the infection includes an infection by hepatitis A virus, hepatitis B virus, hepatitis C virus, hepatitis D virus, hepatitis E virus, hepatitis F virus, HIV virus, influenza virus, herpesvirus (HSV-1, HSV-2), anthrax, chlamydia, pneumococcus bacteria, Japanese encephalitis virus, measles virus, rubella virus, Clostridium tetani, varicella virus, SARS virus, EB virus, papillomavirus, Helicobacter pylori bacteria, rabies virus, West Nile virus, hantavirus, streptococcus, staphylococcus, Bordetella pertussis, Mycobacterium tuberculosis, Plasmodium, or poliomyelitis virus. Examples of the allergy include pollen allergy, various food allergies, and any allergy to a self-antigen.

The composition for enhancing immunogenicity of the present invention can be used as an active ingredient of a vaccine for treatment and/or prevention of cancer in a case where the cancer antigen is used as the antigen. Accordingly, the present invention provides a vaccine containing the composition for enhancing immunogenicity of the present invention, as an active ingredient.

According to a preferred aspect of the present invention, an immuno-stimulating method (in particular, method for enhancing immunogenicity) which comprises administering the composition for enhancing immunogenicity, the medicine, or the vaccine of the present invention, to a (organism) subject, is provided. A method for treating and/or preventing a disease such as cancer, infection, or allergy which comprises administering the composition for enhancing immunogenicity, the medicine, or the vaccine of the present invention, to a (organism) subject, is also provided. The method for activating immune reaction and the method for treating and/or preventing a disease, using the composition for enhancing immunogenicity, the medicine, or the vaccine of the present invention, are not limited. The composition for enhancing immunogenicity, the medicine, or the vaccine may be administered to an organism (subject), or may be contacted with immune competent cells harvested out of an organism (subject). The method for administration to an organism (subject) is not particularly limited, and examples thereof include subcutaneous administration, intracutaneous administration, intramuscular administration, intranasal administration, transpulmonary administration, oral administration, percutaneous administration, sublingual administration, intravaginal administration, intraperitoneal administration, and lymph node administration, and intracutaneous administration or subcutaneous administration is preferable. The organism (subject) for administration may be human or any animal other than human, and is preferably human, or pig, cow, bird, sheep, horse, donkey, goat, camel, dog, cat, ferret, rabbit, monkey, rat, mouse or guinea pig being kept as a livestock animal, a pet animal, or a laboratory animal. The subject may be a subject having a disease such as cancer, infection, or allergy, or may be a subject without a disease such as cancer, infection, or allergy but having a risk of developing the disease.

In a case where the composition for enhancing immunogenicity of the present invention is used as a medicine (including a vaccine), various pharmaceutically useful additives may be compounded to formulate the medicine, and specific examples of such an additive include a buffering agent, an antioxidant, a salt, a polymer, or sugar.

The dose of the particle in the case of using the composition for enhancing immunogenicity of the present invention as a medicine is appropriately set depending on the administration method and the frequency of administration. For example, in a case where the composition for enhancing immunogenicity of the present invention is subcutaneously administered to human, 0.01 to 1,000 mg of the composition, per administration, can be administered as the amount of the particle to which the immuno-stimulating factor is bound.

### Examples

Examples are shown below, but the present invention is not limited by these Examples.

### (Example 1) Synthesis of R848-dextran-PLGA

In the present Example, poly(lactic-co-glycolic acid) (PLGA) was added to dextran to perform condensation reaction, thereby synthesizing a straight-chained block-type polymer. Then, the resulting amphiphilic polymer, dextran-poly(lactic-co-glycolic acid) (dextran-PLGA) was covalently bound to an immuno-stimulating factor, Resiquimod (R848), which was an imidazoquinoline compound and known as a TLR7/8 agonist, thereby synthesizing R848-dextran-poly(lactic-co-glycolic acid) (R848-dextran-PLGA).

### <Synthesis of dextran having primary amino group at reducing terminal (dextran-NH₂)>

Sodium triacetoxyborohydride (203.5 mg) and N-Boc-ethylenediamine (76.1 µl) were added to a dimethylsulfoxide solution of dextran (number-average molecular weight 2,100, Pharmacosmos A/S), having a concentration of 500 mg/2 ml, and then reacted with stirring at 60°C for 91 hours. Next, unreacted N-Boc-ethylenediamine was removed by dialysis using water as an external solution, thereafter freeze-drying was performed, and dextran-NHBoc was synthesized. Deprotection reaction of a Boc terminal group of the resulting dextran-NHBoc (450 mg) (aqueous 35% hydrochloric acid solution (5 ml)/dimethylsulfoxide (5 ml), stirred at ambient temperature for 35 hours) was performed, and then purification by water dialysis was performed, and a polymer powder (dextran-NH₂) was obtained by freeze-drying.

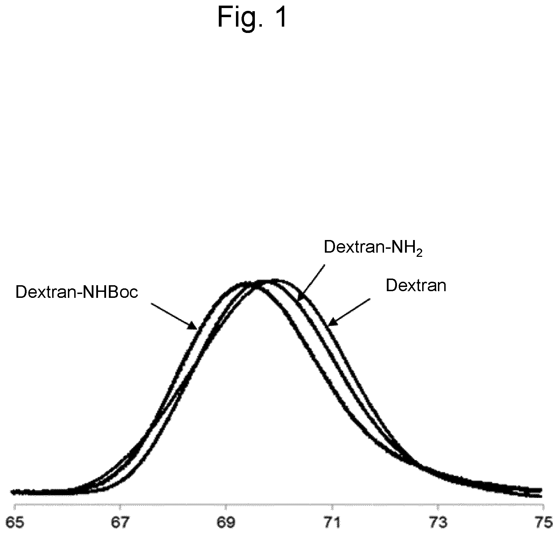

The number-average molecular weight of the dextran-NH₂ was determined by GPC measurement (column: TSK-gel α-5000 × 2, manufactured by Tosoh Corporation, DMF-based solvent, detector: RI, standard product: pullulan) (Table 1, Figure 1: dextran-NH₂). It was confirmed by ¹H-NMR measurement that a primary amino group was introduced to a reducing terminal of dextran.

### <Synthesis of dextran-PLGA>

Poly(lactic-co-glycolic acid) having a heterobifunctional terminal group (COOH-PLGA-OH, FUJIFILM Wako Pure Chemical Corporation, PLGA-5020, number-average molecular weight 8,900) (350 mg) was mixed in a dimethylsulfoxide (0.58 ml) solution of 1-ethyl-3-(3-dimethylaminopropyl)carbodiimide hydrochloride (EDC) (35.5 mg) and N-hydroxysuccinimide (NHS) (21.3 mg), and then reacted at 50°C for 2 hours, thereby converting a carboxyl group at one terminal into an active ester (NHS). To the reaction solution, dextran to which a primary amino group was introduced at a reducing terminal (dextran-NH₂, number-average molecular weight 2,700) (100 mg) was added, and condensation reaction of an active ester (NHS) of NHS-PLGA-OH and the primary amino group of dextran-NH₂ was performed for 110.5 hours. After the reaction, unreacted dextran-NH₂ was removed by water dialysis, and then unreacted NHS-PLGA-OH was removed by ultra-centrifugal purification, to obtain an amphiphilic polymer (1-1).

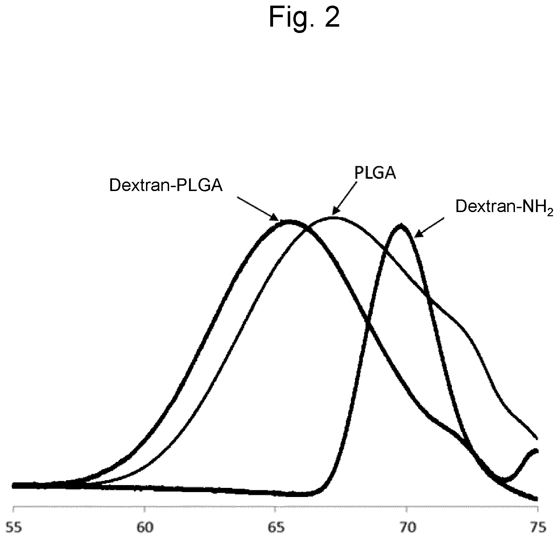

The number-average molecular weight of dextran-PLGA was determined by GPC measurement (column: TSK-gel α-5000 × 2, manufactured by Tosoh Corporation, DMF-based solvent, detector: RI, standard product: pullulan) (Table 1, Figure 2: dextran-PLGA). It was confirmed by ¹H-NMR measurement that condensation reaction progressed.

### <Modification reaction of dextran-PLGA with R848>

### (Synthesis of CASSPy-dextran-PLGA)

A dimethylsulfoxide (0.1 ml) solution of CDI (21.7 mg) was dropped into a dimethylsulfoxide (0.4 ml) solution of dextran-PLGA (100 mg), and then a dimethylsulfoxide (0.1 ml) solution of a heterocrosslinker (cysteamine SS pyridyl; CASSPy) (4.9 mg) obtained by reaction of cysteamine hydrochloride with 2,2'-dipyridyl disulfide was dropped thereto, and the mixture was stirred at room temperature for 2.5 hours. The reaction liquid was placed in a dialysis membrane and subjected to dimethylsulfoxide dialysis followed by water dialysis, the content liquid was centrifuged (9,000 rpm, 4°C, 30 minutes) to remove the supernatant, and the resultant was freeze-dried to obtain a polymer powder.

The number-average molecular weight of CASSPy-dextran-PLGA was determined by GPC measurement (column: TSK-gel α-5000 × 2, manufactured by Tosoh Corporation, DMF-based solvent, detector: RI, standard product: pullulan). The introduction ratio of CASSPy (%) was confirmed by ¹H-NMR measurement.

### (Synthesis of SH-dextran-PLGA)

A dimethylsulfoxide (0.05 ml) solution of dithiothreitol (DTT, 15.5 mg), as a reagent for reducing a disulfide group (SS bond) to an SH group, was added to a dimethylsulfoxide (1.1 ml) solution of CASSPy-dextran-PLGA (108.8 mg), and then reacted at room temperature for 4.5 hours. Next, water dialysis was performed as a purification method for removing an excess amount of DTT, and then a polymer powder was obtained by centrifugation (9000 rpm, 30 minutes, 4°C) and freeze-drying. It was confirmed by ¹H-NMR measurement that pyridyl was deprotected and a thiol group was introduced.

### (Synthesis of R848-dextran-PLGA)

R848 having a maleimide group (Mal-R848; Resiquimod derivative synthesized by adding two carbon atoms to a tert-hydroxyl group in Resiquimod, attaching an amino group thereto, and then binding succinimidyl 4-(N-maleimidomethyl)cyclohexane-1-carboxylate (SMCC) thereto, synthesized on a consignment basis by NARD Institute, Ltd.) (0.008 mmol) was added to a dimethylsulfoxide (1 ml) solution of SH-dextran-PLGA (40 mg), and then reacted at room temperature for 118 hours. Next, dialysis purifications using dimethylsulfoxide and water as external solutions were sequentially performed as a purification method for removing unreacted Mal-R848, and then a powder of a modified amphiphilic polymer (1-2) was obtained by centrifugation (9000 rpm, 30 minutes, 4°C) and freeze-drying.

Dextran-NH₂ having a number-average molecular weight of 1,800 was synthesized according to the above synthesis method of dextran-NH₂. An amphiphilic polymer (2-1) was obtained using dextran-NH₂ (number-average molecular weight of 1,800) and the PLGA-5020 (number-average molecular weight of 8,900) according to the same synthesis method as in the amphiphilic polymer (1-1). Then, a modified amphiphilic polymer (2-2) was obtained according to the same synthesis method as in the modified amphiphilic polymer (1-2). The evaluation results of each of the polymers are shown in Table 1.

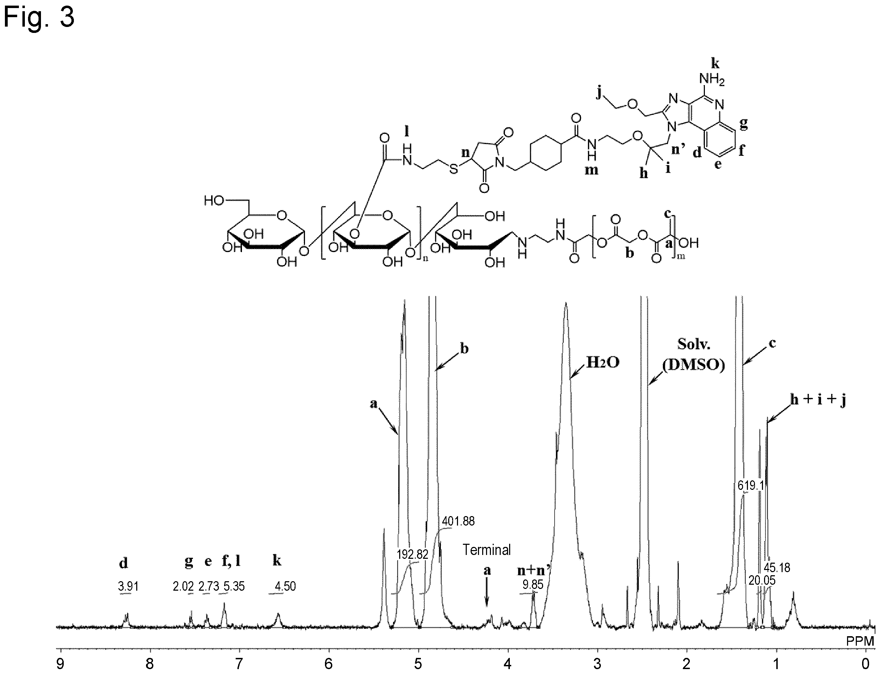

The number-average molecular weights of dextran-PLGA and R848-dextran-PLGA were determined by GPC measurement (column: TSK-gel α-5000 × 2, manufactured by Tosoh Corporation, DMF-based solvent, detector: RI, standard product: pullulan) (Table 1). The introduction ratio of R848 (%) in R848-dextran-PLGA was confirmed by ¹H-NMR measurement (Table 1, Figure 3: R848-dextran-PLGA).

### (Example 2) Synthesis of R848-black yeast glucan-PLGA

In the present Example, poly(lactic-co-glycolic acid) (PLGA) was added to black yeast glucan to perform condensation reaction, thereby synthesizing a straight-chained block-type polymer. Then, the resulting amphiphilic polymer, black yeast glucan-poly(lactic-co-glycolic acid) (black yeast glucan-PLGA) was covalently bound to an immuno-stimulating factor, R848, thereby synthesizing R848-black yeast glucan-poly(lactic-co-glycolic acid) (R848-black yeast glucan-PLGA).

### <Synthesis of black yeast glucan having primary amino group at reducing terminal (black yeast glucan-NH₂)>

5 g of black yeast glucan (DAISO CO.,LTD.) was dissolved in 150 ml of dimethylsulfoxide, and then 5 ml of an aqueous 35% hydrochloric acid solution was added thereto, and the resultant was stirred at 105°C for 20 minutes. The reaction solution was transferred to a dialysis membrane and dialysis was performed in water, followed by freeze-drying to obtain a black yeast glucan hydrolysate (number-average molecular weight 2,400) as a powder.

Sodium triacetoxyborohydride (413.3 mg) and N-Boc-ethylenediamine (246.9 µl) were added to a dimethylsulfoxide solution of a black yeast glucan hydrolysate (number-average molecular weight 2,400) having a concentration of 2.5 g/8 ml, and then reacted with stirring at 55°C for 169.5 hours. Next, unreacted N-Boc-ethylenediamine was removed by dialysis using water as an external solution, and then black yeast glucan-NHBoc was obtained. Next, deprotection reaction of a Boc terminal group of black yeast glucan-NHBoc (1.19 g) (aqueous 35% hydrochloric acid solution (14.3 ml)/dimethylsulfoxide (24 ml), stirred at ambient temperature for 2 hours) was performed, and then purification by water dialysis was performed, and a polymer powder (black yeast glucan-NH₂) was obtained by freeze-drying.

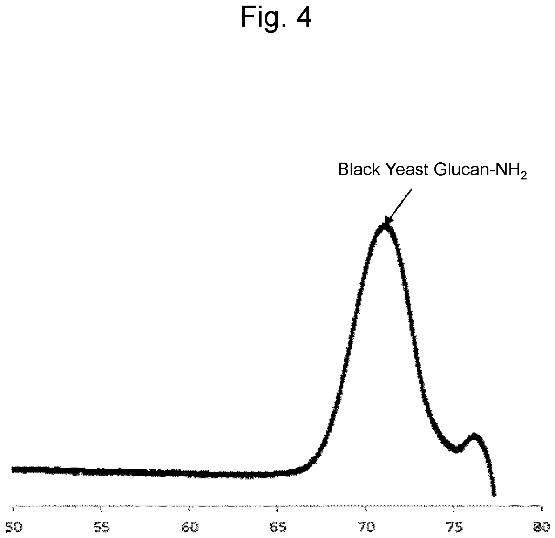

The number-average molecular weight of the black yeast glucan-NH₂ was determined by GPC measurement (column: TSK-gel α-5000 × 2, manufactured by Tosoh Corporation, DMF-based solvent, detector: RI, standard product: pullulan) (Table 1, Figure 4: black yeast glucan-NH₂). It was confirmed by ¹H-NMR measurement that a primary amino group was introduced into a reducing terminal of black yeast glucan.

### <Synthesis of black yeast glucan-PLGA>

Poly(lactic-co-glycolic acid) having a heterobifunctional terminal group (COOH-PLGA-OH, FUJIFILM Wako Pure Chemical Corporation, PLGA-5020, number-average molecular weight 8,900) (4.462 g) was mixed in a dimethylsulfoxide (2.5 ml) solution of EDC (479.25 mg) and NHS (287.73 mg), and then reacted at 50°C for approximately 18 hours, thereby converting a carboxyl group at the α-terminal into an active ester (NHS). To the reaction solution, black yeast glucan having a primary amino group at the ω-terminal (black yeast glucan-NH₂, number-average molecular weight 1,500) (749 mg) was added, and condensation reaction (270.5 hours) of an active ester (NHS) of NHS-PLGA-OH and the primary amino group of black yeast glucan-NH₂ was performed. After the reaction, unreacted black yeast glucan-NH₂ was removed by water dialysis, and then unreacted NHS-PLGA-OH was removed by ultra-centrifugal purification to obtain a non-modified amphiphilic polymer (3-1).

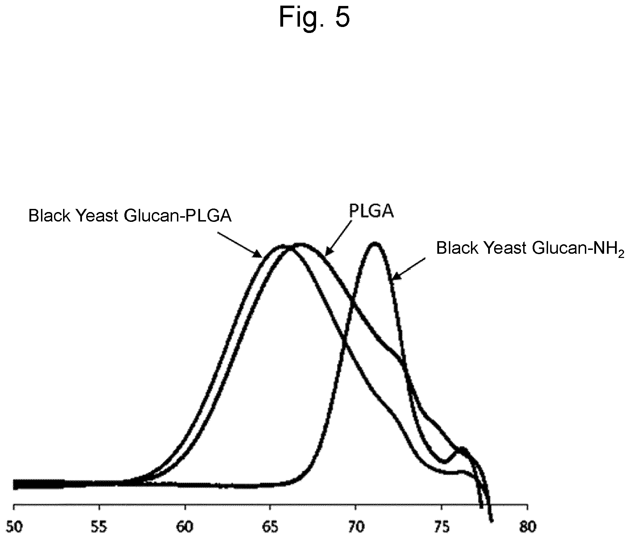

The number-average molecular weight of black yeast glucan-PLGA was determined by GPC measurement (column: TSK-gel α-5000 × 2, manufactured by Tosoh Corporation, DMF-based solvent, detector: RI, standard product: pullulan) (Table 1, Figure 5: black yeast glucan-PLGA). It was confirmed by ¹H-NMR measurement that condensation reaction progressed.

### <Modification reaction of black yeast glucan-PLGA with R848>

### (Synthesis of CASSPy-black yeast glucan-PLGA)

A dimethylsulfoxide (0.1 ml) solution of CDI (26 mg) was dropped into a dimethylsulfoxide (0.5 ml) solution of black yeast glucan-PLGA (polymer (3-1), 200 mg), and stirred at room temperature for 22 hours. Next, a dimethylsulfoxide (0.15 ml) solution of a heterocrosslinker (cysteamine SS pyridyl; CASSPy) (29.1 mg) obtained by reaction of cysteamine hydrochloride with 2,2'-dipyridyl disulfide was dropped thereto, and the mixture was stirred at room temperature for 65.5 hours. The reaction liquid was placed in a dialysis membrane and subjected to dimethylsulfoxide dialysis followed by water dialysis, the content liquid was centrifuged (9,000 rpm, 4°C, 30 minutes) to remove the supernatant, and the resultant was freeze-dried to obtain a polymer powder.

The number-average molecular weight of CASSPy-black yeast glucan-PLGA was determined by GPC measurement (column: TSK-gel α-5000 × 2, manufactured by Tosoh Corporation, DMF-based solvent, detector: RI, standard product: pullulan). The introduction ratio of CASSPy (%) was confirmed by ¹H-NMR measurement.

### (Synthesis of SH-black yeast glucan-PLGA)

A dimethylsulfoxide (0.1 ml) solution of dithiothreitol (DTT, 40 mg), as a reagent for reducing a disulfide group (SS bond) to an SH group, was added to a dimethylsulfoxide (1 ml) solution of CASSPy-black yeast glucan-PLGA (150 mg), and then reacted at room temperature for 4 hours. Next, water dialysis was performed as a purification method for removing an excess amount of DTT, and then a polymer powder was obtained by centrifugation (9000 rpm, 30 minutes, 4°C) and freeze-drying. It was confirmed by ¹H-NMR measurement that pyridyl was deprotected and a thiol group was introduced.

### (Synthesis of R848-black yeast glucan-PLGA)

R848 having a maleimide group (Mal-R848, synthesized on a consignment basis by NARD Institute, Ltd.) (0.024 mmol) was added to a dimethylsulfoxide (0.75 ml) solution of SH-black yeast glucan-PLGA (109.5 mg), and then reacted at room temperature for approximately 69 hours. Next, dialysis purifications using dimethylsulfoxide and water as external solutions were sequentially performed as a purification method for removing unreacted Mal-R848, and then a powder of a modified amphiphilic polymer (3-2) was obtained by centrifugation (9000 rpm, 30 minutes, 4°C) and freeze-drying.

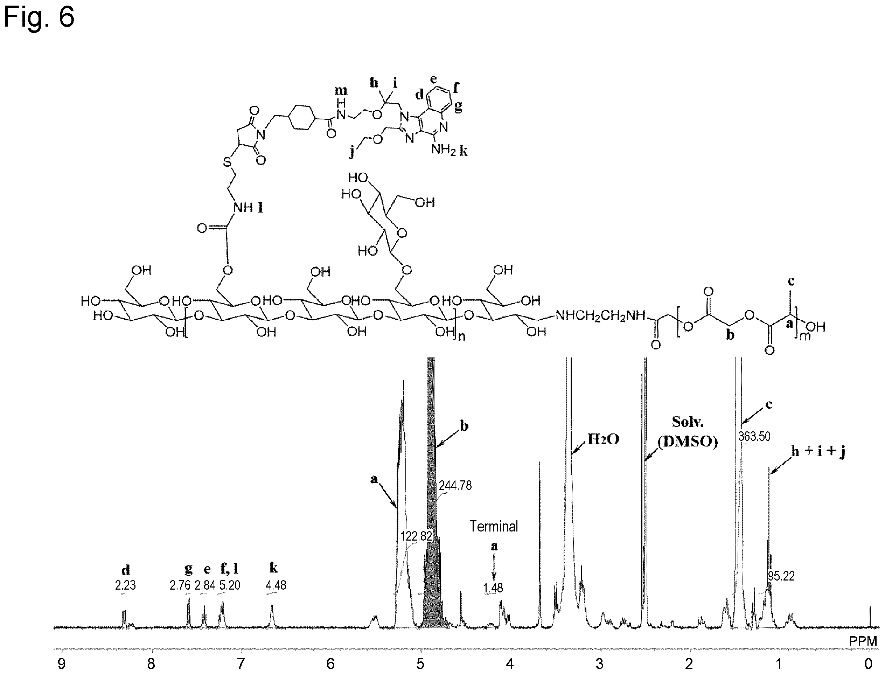

The number-average molecular weight of R848-black yeast glucan-PLGA was determined by GPC measurement (column: TSK-gel α-5000 × 2, manufactured by Tosoh Corporation, DMF-based solvent, detector: RI, standard product: pullulan) (Table 1). The introduction ratio of R848 (%) in R848-black yeast glucan-PLGA was confirmed by ¹H-NMR measurement (Table 1, Figure 6: R848-black yeast glucan-PLGA).

Modified amphiphilic polymers having different amounts of modification with R848 to one amphiphilic polymer molecule were synthesized by quantitatively controlling the amount of introduction of CASSPy to hydroxyl groups in a polysaccharide main chain, according to the same method. For example, while the amount of modification with R848 to one molecule of the modified amphiphilic polymer (3-2) was 1.5 molecules, a modified amphiphilic polymer (3-4) was obtained by controlling the amount of introduction of CASSPy to a larger amount, and a modified amphiphilic polymer (3-3) was obtained by controlling the amount of introduction of CASSPy to a smaller amount, using the same amphiphilic polymer (3-1).

Black yeast glucan-NH₂ having a number-average molecular weight of 1,200 or 2,300, and curdlan-NH₂ having a number-average molecular weight of 3,500 were synthesized according to the above synthesis method of black yeast glucan-NH₂. Then, an amphiphilic polymer (4-1) and a modified amphiphilic polymer (4-2) were obtained by using black yeast glucan-NH₂ (number-average molecular weight 1,200) and PLGA-5020 (number-average molecular weight 8,900); an amphiphilic polymer (5-1) was obtained by using black yeast glucan-NH₂ (number-average molecular weight 2,300) and PLGA-5020 (number-average molecular weight 8,900); and an amphiphilic polymer (6-1) and a modified amphiphilic polymer (6-2) were obtained by using curdlan-NH₂ (number-average molecular weight 3,500) and PLGA-5020 (number-average molecular weight 8,900), according to the same synthesis method as in the amphiphilic polymer (3-1) and the modified amphiphilic polymer (3-2). The evaluation results of each of the polymers are shown in Table 1.

**[Table 1]**

| Results of analysis on various amphiphilic polymers (polysaccharide-PLGA) | | | | | |
|---|---|---|---|---|---|
| Amphiphilic polymer | Number-average molecular weight | Hydrophilic polysaccharide portion | Number-average molecular weight of hydrophilic polysaccharide | Number-average molecular weight of PLGA | Amount of modification with R848 |
| (1-1) | 14500 | Dextran | 2700 | 11800 | - |
| (1-2) | 8700 | Dextran | 2700 | 6000 | 2.0 molecules |
| (2-1) | 13400 | Dextran | 1800 | 11600 | - |
| (2-2) | 2400 | Dextran | 1800 | 600 | 5.6 molecules |
| (3-1) | 12200 | Black yeast glucan | 1500 | 10700 | - |
| (3-2) | 3100 | Black yeast glucan | 1500 | 1600 | 1.5 molecules |
| (3-3) | 4500 | Black yeast glucan | 1500 | 3000 | 1.0 molecule |
| (3-4) | 2200 | Black yeast glucan | 1500 | 700 | 3.5 molecules |
| (4-1) | 12400 | Black yeast glucan | 1200 | 11200 | - |
| (4-2) | 2200 | Black yeast glucan | 1200 | 1000 | 7.5 molecules |
| (5-1) | 9900 | Black yeast glucan | 2300 | 7600 | - |
| (6-1) | 12700 | Curdlan | 3500 | 9200 | - |
| (6-2) | 10900 | Curdlan | 3500 | 7400 | 7.3 molecules |

### (Example 3) Preparation of particles (OVA-containing R848-modified dextran-PLGA particle (1), OVA-containing R848-modified black yeast glucan-PLGA particles (2) to (9) and OVA-containing R848-modified curdlan-PLGA particle (10), and comparative particles: OVA-containing dextran-PLGA particle (11) and OVA-containing black yeast glucan-PLGA particle (12)), by S/O/W emulsion method

### <Preparation of OVA-containing R848-modified dextran-PLGA particle>

A polymer mixture powder (30 mg) of R848-dextran-PLGA (Table 1, amphiphilic polymer (1-2)) (6 mg) and dextran-PLGA (Table 1, amphiphilic polymer (1-1)) (24 mg) was dissolved in 1.2 ml of dimethyl carbonate and 133 µl of tert-butanol to prepare a polymer solution. To the polymer solution, 0.3 ml of an aqueous 0.5% (w/v) OVA (ovalbumin, Sigma-Aldrich Co. LLC) solution was dropped, and the resultant was stirred with a mixer (PT2100S manufactured by Polytron) at 11,000 rpm for 1 minute, thereby producing a W/O emulsion solution. The W/O emulsion solution was preliminarily frozen by liquid nitrogen, and then freeze-dried with a freeze-dryer (TOKYO RIKAKIKAI CO., LTD., FD-1000) at a trap cooling temperature of -45°C and a degree of vacuum of 20 Pa for 4 hours. The resulting solid content was dispersed in 3 ml of ethyl acetate, thereby preparing a S/O suspension solution. The S/O suspension solution was dropped in 12 ml of an aqueous 1% (w/v) polyvinyl alcohol solution, and stirred with a mixer (Silverson Nippon Limited, L5M-A) at 5,000 rpm for 3 minutes, thereby preparing a S/O/W-type emulsion solution. Ethyl acetate was removed from the S/O/W-type emulsion solution by drying in liquid to obtain a particle suspension liquid. The suspension liquid was transferred to a 50-ml tube, and a particle was precipitated by centrifugation at 3,000 g for 30 minutes. After the supernatant was removed, the particle was re-suspended in 50 ml of distilled water, and washed by re-precipitation of the particle by centrifugation in the above conditions. After the washing operation was repeated once more and the supernatant was removed, the particle was suspended in 2.4 ml of an aqueous solution containing 5% (w/v) mannitol and 0.1% (w/v) polysorbate 80. The suspension liquid was filtrated with a mesh filter (1 µm), and preliminarily frozen by liquid nitrogen, and then freeze-dried with a freeze-dryer at a trap cooling temperature of -45°C and a degree of vacuum of 20 Pa for 12 hours to obtain an OVA-containing R848-modified dextran-PLGA particle (1).

### <Preparation of OVA-containing R848-modified black yeast glucan-PLGA particle>

A polymer mixture powder (50 mg) of R848-black yeast glucan-PLGA (Table 1, modified amphiphilic polymer (3-2)) (10 mg) and black yeast glucan-PLGA (Table 1, non-modified amphiphilic polymer (3-1)) (40 mg) was dissolved in a mixed solution of 0.9 ml of dimethyl carbonate and 100 µl of tert-butanol to prepare a polymer solution. To the polymer solution, 0.5 ml of an aqueous 0.5% (w/v) OVA (ovalbumin, Sigma-Aldrich Co. LLC)) solution was dropped, and the resultant was stirred with a mixer (PT2100S manufactured by Polytron) at 11,000 rpm for 1 minute, thereby producing a W/O emulsion solution. The W/O emulsion solution was preliminarily frozen by liquid nitrogen, and then freeze-dried with a freeze-dryer (TOKYO RIKAKIKAI CO., LTD., FD-1000) at a trap cooling temperature of -45°C and a degree of vacuum of 20 Pa for 12 hours. The resulting solid content was dispersed in 5 ml of ethyl acetate, thereby preparing a S/O suspension solution. The S/O suspension solution was dropped in 20 ml of an aqueous 1% (w/v) polyvinyl alcohol solution, and stirred with a mixer (Silverson Nippon Limited, L5M-A) at 6,000 rpm for 5 minutes, thereby preparing a S/O/W-type emulsion solution. Ethyl acetate was removed from the S/O/W-type emulsion solution by drying in liquid to obtain a particle suspension liquid. The suspension liquid was transferred to a 50-ml tube, and a particle was precipitated by centrifugation at 8,000 rpm for 10 minutes. After the supernatant was removed, the particle was re-suspended in 25 ml of distilled water, and washed by re-precipitation of the particle by centrifugation in the above conditions. After the washing operation was repeated once more and the supernatant was removed, the particle was suspended in 4 ml of an aqueous solution containing 5% (w/v) mannitol and 0.1% (w/v) polysorbate 80. The suspension liquid was preliminarily frozen by liquid nitrogen, and then freeze-dried with a freeze-dryer at a trap cooling temperature of -45°C and a degree of vacuum of 20 Pa for 12 hours to obtain an OVA-containing R848-modified black yeast glucan-PLGA particle (2). The same method was performed to obtain OVA-containing R848-modified black yeast glucan-PLGA particles (3) to (9) using R848-black yeast glucan-PLGA and black yeast glucan-PLGA (Table 1, amphiphilic polymers (3-1) to (3-4), (4-1), and (4-2)) as base materials at various ratios.

### <Preparation of OVA-containing R848-modified curdlan-PLGA particle>

An OVA-containing R848-modified curdlan-PLGA particle (10) was obtained by the same method as described above, using a polymer mixture powder of R848-curdlan-PLGA (Table 1, modified amphiphilic polymer (6-2)) and curdlan-PLGA (Table 1, non-modified amphiphilic polymer (6-1)).

### <Preparation of Comparative particles: OVA-containing dextran-PLGA particle and OVA-containing black yeast glucan-PLGA particle>

An OVA-containing dextran-PLGA particle (11) was obtained by the same method as described above, using a polymer powder of dextran-PLGA alone (Table 1, non-modified amphiphilic polymer (1-1)).

An OVA-containing black yeast glucan-PLGA particle (12) was obtained by the same method as described above, using a polymer powder of black yeast glucan-PLGA alone (Table 1, non-modified amphiphilic polymer (3-1)).

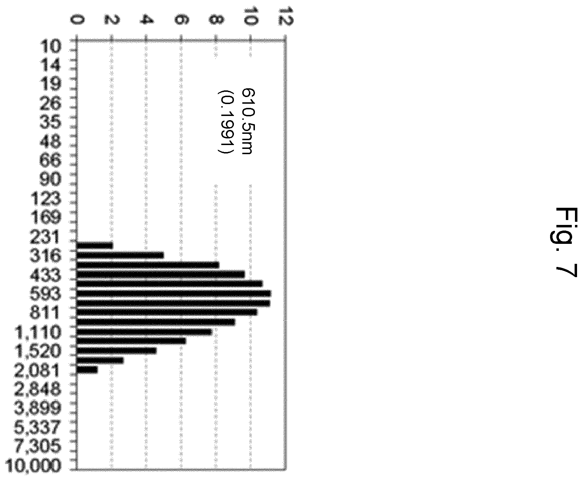

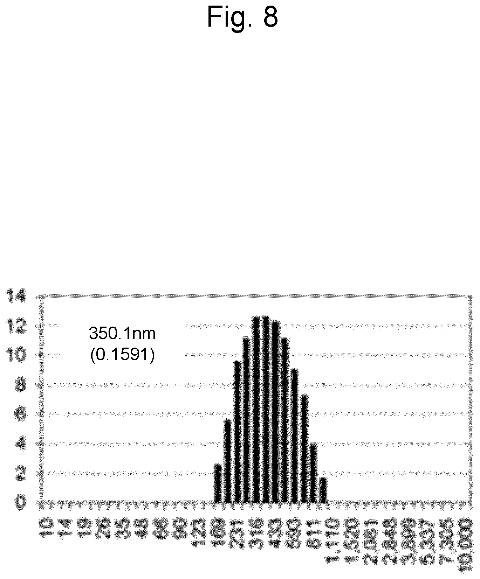

The evaluation results of each of the particles are shown in Table 2. The average particle size was calculated with a dynamic light scattering apparatus (ELS-Z manufactured by OTSUKA ELECTRONICS CO., LTD.) by a cumulant method (Table 2, Figure 7: OVA-containing R848-modified dextran-PLGA particle, Figure 8: OVA-containing R848-modified black yeast glucan-PLGA particle). The content ratio (w/w) of the OVA antigen was determined by extracting the antigen from the particle using an organic solvent, and subjecting the antigen extracted, to gel electrophoresis with a gel electrophoresis apparatus (TEFCO) and then staining with a colloidal CBB staining kit (TEFCO).

**[Table 2]**

| Results of analysis on various OVA-containing particles | | | | |
|---|---|---|---|---|
| Particle | Amphiphilic polymer (weight ratio) | Average particle size (nm) | Content ratio of antigen (%) | Amount of introduction of R848 per mg of particle powder (pmol) |
| (1) | R848-dextran-PLGA (20%) | 610.5 | 3.90 | 0.00916 |
| | Dextran-PLGA (80%) | | | |
| (2) | R848-black yeast glucan-PLGA (20%) | 350.1 | 4.50 | 0.01956 |
| | Black yeast glucan-PLGA (80%) | | | |
| (3) | R848-black yeast glucan-PLGA (20%) | 740.1 | 6.92 | 0.06384 |
| | Black yeast glucan-PLGA (80%) | | | |
| (4) | R848-black yeast glucan-PLGA (5%) Black yeast glucan-PLGA (95%) | 622.2 | 4.25 | 0.00978 |
| (5) | R848-black yeast glucan-PLGA (10%) Black yeast glucan-PLGA (90%) | 620.2 | 4.35 | 0.00489 |
| (6) | R848-black yeast glucan-PLGA (20%) Black yeast glucan-PLGA (80%) | 598.5 | 3.40 | 0.01956 |
| (7) | R848-black yeast glucan-PLGA (20%) Black yeast glucan-PLGA (80%) | 661.7 | 4.96 | 0.01956 |
| (8) | R848-black yeast glucan-PLGA (50%) Black yeast glucan-PLGA (50%) | 691.9 | 4.65 | 0.04891 |
| (9) | R848-black yeast glucan-PLGA (100%) | 744.7 | 3.85 | 0.09782 |
| (10) | R848-curdlan-PLGA (80%) Curdlan-PLGA (20%) | 941.5 | 0.77 | 0.10680 |
| Comparative particle (11) | Dextran-PLGA (100%) | 629.7 | 3.49 | - |
| Comparative particle (12) | Black yeast glucan-PLGA (100%) | 408.3 | 4.30 | - |

### (Example 4) Preparation of particles (TRP2-containing R848-modified black yeast glucan-PLGA particles (13) to (16), and comparative particle: TRP2-containing black yeast glucan-PLGA particle (17)), by S/O/W emulsion method

The following particles were produced using an epitope peptide of tyrosinase-associated protein 2 (TRP2) known as a cancer antigen, particularly a melanoma antigen, specifically, a common epitope peptide for a human-derived TRP2 amino acid sequence (UniProt Accession Number: P40126) and a mouse-derived TRP2 amino acid sequence (UniProt Accession Number: P29812), i.e., the polypeptide of a region of positions 180 to 188 of the above amino acid sequences.

### <Preparation of TRP2-containing R848-modified black yeast glucan-PLGA particle>

4 ml of dimethyl carbonate (comprising 20% t-butanol and 0.05% span 80) was added to a polymer mixture powder (100 mg) of R848-black yeast glucan-PLGA (Table 1, modified amphiphilic polymer (4-2)) and black yeast glucan-PLGA (Table 1, non-modified amphiphilic polymer (4-1)), and the powder was dissolved by a compact mixer (11,000, 1 minute). To the resulting polymer solution, 1 ml of an aqueous acetonitrile solution dissolving the TRP2 epitope peptide (Greiner Bio-One International GmbH) (75%, 0.1% trifluoroacetic acid (TFA); TRP2 peptide: 1 mg/ml) was added, and then 1 ml of distilled water was added. Then, they were emulsified by a compact mixer (19,000 rpm, 1 minute). The resulting emulsified liquid was freeze-dried, and 10 ml of ethyl acetate was added to the resulting powder, and the powder was dispersed by stirring with a stirrer (300 rpm, 1 minute). Then, 40 ml of an aqueous polyvinyl alcohol (PVA) solution (Nippon Synthetic Chemical Industry Co., Ltd., 1% EG-05P) was added thereto, and roughly emulsified by stirring with a stirrer (500 rpm, 1 minute). This liquid was further stirred by a mixer (5,000 rpm, 10 minutes) and emulsified. Next, stirring with a stirrer (100 rpm, 10 minutes) was performed for degassing, and then the organic solvent was removed by an evaporator. The remaining liquid was filtered with a cell strainer (40 µm), cooled with ice for 30 minutes, and then centrifuged (3,000 g, 4°C, 30 minutes) to remove the supernatant. Then, 50 ml of an aqueous 0.1% Tween 80 solution was added thereto, and dispersion was performed by vortexing, and then centrifugation (4,000 g, 4°C, 30 minutes) was performed to remove the supernatant. 8 ml of an aqueous mannitol solution (aqueous 5% mannitol, 0.1% PVA solution) was added thereto as an excipient, and dispersion was performed by vortexing. The resultant was filtered with a mesh filter (1 µm) under reduced pressure and freeze-dried overnight to obtain a TRP2-containing R848-modified black yeast glucan-PLGA particle (14). TRP2-containing R848-modified black yeast glucan-PLGA particles (13), (15), and (16) were obtained by the same method except that R848-modified black yeast glucan-PLGA (Table 1, amphiphilic polymer 4-2) and black yeast glucan-PLGA (Table 1, amphiphilic polymer 4-1) were used at different weight ratios.

### <Preparation of Comparative particle: TRP2-containing black yeast glucan-PLGA particle>

A TRP2-containing black yeast glucan-PLGA particle (17) was obtained by the same method as described above, using a polymer powder of black yeast glucan-PLGA alone (Table 1, non-modified amphiphilic polymer (5-1)).

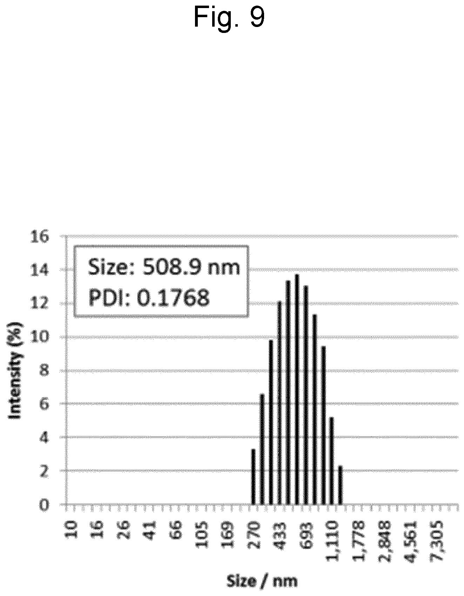

The evaluation results of each of the particles are shown in Table 3. The average particle size was calculated with a dynamic light scattering apparatus (ELS-Z manufactured by OTSUKA ELECTRONICS CO., LTD.) by a cumulant method (Table 3, Figure 9: TRP2-containing R848-modified black yeast glucan-PLGA particle).

The content ratio (w/w) of TRP2 peptide (antigen) was evaluated by HPLC. 1 ml of acetone was added to 5 mg of the particle powder, and the powder was dispersed by vortexing for 30 seconds and an ultrasonic treatment for 5 minutes, and the supernatant was removed after centrifugation (13,000 rpm, 4°C, 5 minutes). 1 ml of acetone was further added to the resulting precipitate, and the precipitate was dispersed by vortexing for 30 seconds, and the supernatant was removed after centrifugation (13,000 rpm, 4°C, 5 minutes) (twice in total). The resultant was dried by a centrifugal evaporator at 30°C for 30 minutes. 200 µl of an aqueous 50% acetonitrile solution (containing 0.1% TFA) was added to the extracted component after drying, and the component was dissolved by vortexing for 3 seconds and an ultrasonic treatment for 5 minutes. 20 µl of acetylated tryptophan (10 µg/ml in an aqueous 1% dimethylsulfoxide, 0.1% TFA solution) as an internal standard was added thereto, and the supernatant was collected after centrifugation (13,000 rpm, 4°C, 5 minutes). The supernatant was injected into HPLC (SCL-10A manufactured by Shimadzu Corporation, column: YMC-pack ODS-AM [AM12S05-1506WT]), and gradient elution was performed with gradually increasing acetonitrile concentration of an eluent. Detection was performed based on ultraviolet-visible absorption (220 nm), and the ratio of the peak area of peptide to the peak area of the internal standard was calculated. The content ratio of TRP2 peptide antigen was determined, assuming the measurement result for TRP2 peptide + mannitol powder sample subjected to the same treatment as described above as a content ratio of 100%.

**[Table 3]**

| Results of analysis on various TRP2-containing particles | | | | |
|---|---|---|---|---|
| Particle | Amphiphilic polymer (weight ratio) | Average particle size (nm) | Content ratio of antigen (%) | Amount of introduction of R848 per mg of particle powder (µmol) |
| (13) | R848-black yeast glucan-PLGA (10%) Black yeast glucan-PLGA (90%) | 462.4 | 0.74 | 0.0612 |
| (14) | R848-black yeast glucan-PLGA (20%) Black yeast glucan-PLGA (80%) | 508.9 | 0.68 | 0.1363 |
| (15) | R848-black yeast glucan-PLGA (35%) Black yeast glucan-PLGA (75%) | 553.1 | 0.70 | 0.0157 |
| (16) | R848-black yeast glucan-PLGA (50%) Black yeast glucan-PLGA (50%) | 434.4 | 0.92 | 0.2280 |
| Comparative particle (17) | Black yeast glucan-PLGA (100%) | 491.0 | 0.82 | - |

### (Reference Example 1) Induction of mouse bone marrow-derived dendritic cells

C57BL/6 female mice (manufactured by Japan SLC, Inc.) were each euthanized by cervical dislocation, and then the femur was collected. Both ends of the femur were cut by scissors, a RPMI1640 medium (hereinafter, RPMI medium) containing 10% fetal bovine serum (FBS, Sigma-Aldrich Co. LLC), 100 units/ml of penicillin, and 100 µg/ml of streptomycin (NACALAI TESQUE, INC.) was injected into the femur with an injector, and then bone-marrow cells were collected. The cells were precipitated by centrifugation at 410 g for 5 minutes to remove the supernatant. The cells collected were suspended in 1 ml of a buffer for hemolysis (BD Bioscience), and then left to stand at room temperature for 5 minutes to be hemolyzed. 10 ml of the RPMI medium was added to a cell suspension liquid after hemolysis, the cells were precipitated by centrifugation at 1,500 rpm for 5 minutes, and the supernatant was removed. The cells were suspended in a RPMI medium (hereinafter, culture medium) containing GM-CSF, and then seeded to a 6-well plate (Corning Incorporated, Flat Bottom Tissue culture Treated, Polystyrene). The plate to which the cells were seeded was incubated in a CO₂ incubator (PHC Corporation) set to conditions of 5% CO₂, 37°C, and a humidity of 100%. The culture medium was exchanged on day 2 and day 5, and floating cells were aspirated on day 6 of the culturing, and then cells were strongly suspended with a micropipette, thereby collecting cells slightly bound to the plate, namely, only induced dendritic cells. The dendritic cells collected were suspended in "CELLBANKER2" (NIPPON ZENYAKU KOGYO CO., LTD.), and stored at -150°C until use.

### (Example 5) In vitro stimulation test using mouse bone marrow-derived dendritic cells (BMDC) (TRP2-containing R848-modified black yeast glucan-PLGA particle (14))

### <Method>

The dendritic cells obtained in Reference Example 1 were seeded at 1 × 10⁵ cells per well to a 96-well plate together with a culture medium, and then the TRP2-containing R848-modified black yeast glucan-PLGA particle (14) obtained in Example 4 was added thereto at 0.1 mg/ml. The plate to which the cells were seeded was incubated in a CO₂ incubator for 18 hours, and the supernatant was collected. The amount of TNF-α in the supernatant was measured using "Mouse TNF-α ELISA developing kit" (Mabtech AB) according to an attached protocol. In Comparative Examples, 0.1 µg/ml of LPS (positive control); an antigen (TRP2 peptide); 0.1 mg/ml of the TRP2-containing black yeast glucan-PLGA particle (17); or a mixture of 0.1 mg/ml of the TRP2-containing black yeast glucan-PLGA particle (17) and R848, was used. In Comparative Examples, the antigen and R848 were added in amounts equal to their contents in 0.1 mg/ml of the TRP2-containing R848-modified black yeast glucan-PLGA particle (14).

### <Results>

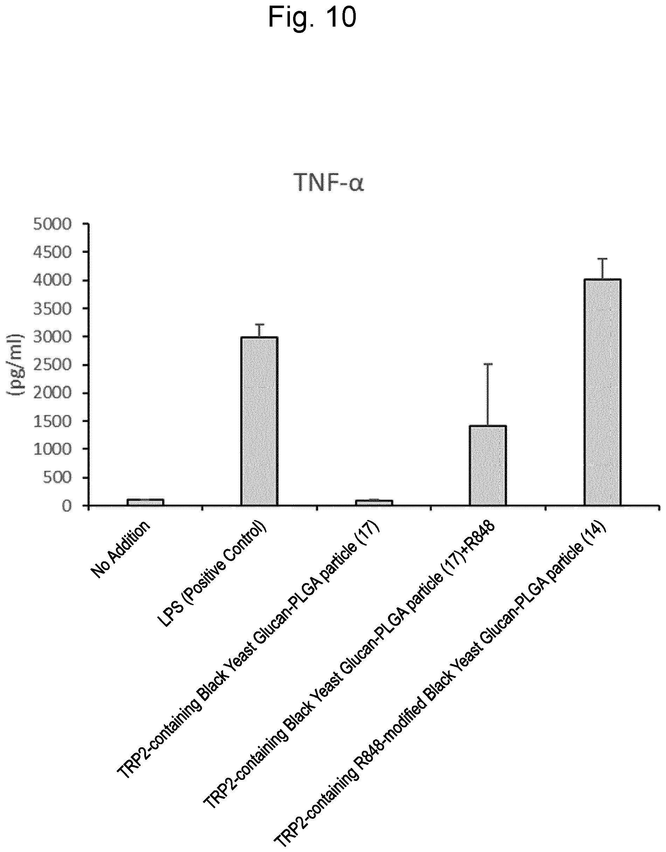

The amount of TNF-α in the supernatant is shown in Figure 10. In the case of using the TRP2-containing R848-modified black yeast glucan-PLGA particle (14), the amount of production of TNF-α was higher than those of the TRP2-containing black yeast glucan-PLGA particle (17) and the mixture of the TRP2-containing black yeast glucan-PLGA particle (17) and R848. Thus, it was revealed that binding R848 to the TRP2-containing black yeast glucan-PLGA particle leads to more strong activation of dendritic cells.

### (Example 6) In vitro stimulation test 2 using mouse bone marrow-derived dendritic cells (BMDC) (OVA-containing R848-modified dextran-PLGA particle (1) and OVA-containing R848-modified black yeast glucan-PLGA particle (2))

### <Method>

The dendritic cells obtained in Reference Example 1 were seeded at 2 × 10⁵ cells per well to a 48-well plate together with a culture medium, and then the OVA-containing R848-modified black yeast glucan-PLGA particle (2) or the OVA-containing R848-modified dextran-PLGA particle (1) obtained in Example 3 was each added at 0.1 mg/ml. A group to which OVA alone was added in the same amount as in the particle, was set as Comparative Example. Then, the cells were cultured in a CO₂ incubator for 18 hours, and the supernatant was removed. Then, 0.5 mM EDTA was added thereto to treat the cells for 5 minutes, and the cells were exfoliated and collected with a micropipette. The cell suspension liquid collected was replaced to be in a phosphate buffer solution containing 1% FBS. An APC-labeled anti-CD86 antibody, a FITC-labeled anti-CD11c antibody, and an APC-Cy7-labeled anti-MHCII antibody (all were manufactured by BD Bioscience) were added thereto, and left to stand at 4°C for 30 minutes, thereby performing antibody labeling reaction. After termination of the antibody labeling reaction, the expression level of an activation marker (CD86) of dendritic cells (CD11c-positive MHCII-positive cells) was evaluated based on the mean fluorescence intensity (MFI) using flow cytometry. In Comparative Examples, OVA alone (negative control) was added in the same concentration, and the expression level of an activation marker was compared in the same way.

### <Results>

As the result of analyzing the MFI as an indicator of the expression level of CD86, the OVA-containing R848-modified black yeast glucan-PLGA particle (2) exhibited an expression intensity 2.39 times higher than that of the OVA alone group. Furthermore, the OVA-containing R848-modified dextran-PLGA particle (1) exhibited a fluorescence intensity 3.11 times higher than that of OVA alone group. Thus, it was revealed that binding R848 to the dextran-PLGA particle also leads to remarkably high dendritic cell activation capacity, as with the black yeast glucan-PLGA particle.

### (Example 7) In vitro stimulation test 3 using mouse bone marrow-derived dendritic cells (BMDC) (OVA-containing R848-modified black yeast glucan-PLGA particles (5), (7), (8), and (9))

### <Method>

The OVA-containing R848-modified black yeast glucan-PLGA particles (5), (7), (8), and (9) obtained in Example 3 were evaluated using the dendritic cells obtained in Reference Example 1, according to the same method as in Example 6. The OVA-containing R848-modified black yeast glucan-PLGA particles (5), (7), (8), and (9) were each added at 0.1 mg/ml. In Comparative Example, OVA alone (negative control) was added at the same concentration.

### <Results>

As the result of analyzing the MFI as an indicator of the expression level of CD86 in the same manner as in Example 6, the OVA-containing R848-modified black yeast glucan-PLGA particles (5), (7), (8), and (9) respectively exhibited fluorescence intensities 4.50 times, 5.77 times, 5.55 times, and 5.35 times higher than that of the OVA alone group that was Comparative Example. Thus, it was revealed that, when the ratio of the weight of the modified amphiphilic polymer with respect to the total weight of the modified amphiphilic polymer and the non-modified amphiphilic polymer is any of 10 to 100%, equally high dendritic cell activation capacity is exhibited.

### Industrial Applicability

The composition for enhancing immunogenicity of the present invention can be used as a medicine, particularly as a vaccine for treatment and/or prevention of infection, cancer, or the like.

All publications, patents, and patent applications herein cited are to be herein incorporated by reference as they are.

## Claims

1. A composition for enhancing immunogenicity, containing, as an active ingredient, a particle comprising a modified amphiphilic polymer and an antigen, wherein the modified amphiphilic polymer is an amphiphilic polymer in which a hydrophobic segment is poly(hydroxy acid) and a hydrophilic segment is polysaccharide and to which an immuno-stimulating factor is bound.

2. The composition for enhancing immunogenicity according to claim 1, wherein the composition does not comprise any lipid as a constituent of the particle other than the antigen.

3. The composition for enhancing immunogenicity according to claim 1 or 2, wherein the particle further comprises a non-modified amphiphilic polymer, wherein the non-modified amphiphilic polymer is an amphiphilic polymer in which a hydrophobic segment is poly(hydroxy acid) and a hydrophilic segment is polysaccharide and to which no immuno-stimulating factor is bound.

4. The composition for enhancing immunogenicity according to any one of claims 1 to 3, wherein the polysaccharide is dextran, β-glucan, mannan, chitin, chitosan, gellan gum, alginic acid, hyaluronic acid, or pullulan.

5. The composition for enhancing immunogenicity according to claim 4, wherein the β-glucan is a polymer of glucose linked by one or more β-1,3 bonds and/or one or more β-1,6 bonds.

6. The composition for enhancing immunogenicity according to claim 4 or 5, wherein the β-glucan is black yeast glucan, curdlan, pachyman, laminaran, lichenan, schizophyllan, lentinan, scleroglucan, or pachymaran.

7. The composition for enhancing immunogenicity according to any one of claims 1 to 6, wherein the poly(hydroxy acid) is poly(lactic-co-glycolic acid), polylactic acid, or polyglycolic acid.

8. The composition for enhancing immunogenicity according to any one of claims 1 to 7, wherein binding between the amphiphilic polymer and the immuno-stimulating factor in the modified amphiphilic polymer is covalent binding.

9. The composition for enhancing immunogenicity according to any one of claims 1 to 8, wherein a portion to which the immuno-stimulating factor is bound in the modified amphiphilic polymer is the hydrophilic segment.

10. The composition for enhancing immunogenicity according to any one of claims 1 to 9, wherein the immuno-stimulating factor is a ligand or agonist binding to a Toll-like receptor (TLR), a NOD-like receptor (NLR), a RIG-like receptor or a C-type lectin receptor (CLR), or a stimulator of interferon gene (STING).

11. The composition for enhancing immunogenicity according to claim 10, wherein the ligand or agonist binding to a Toll-like receptor (TLR) is a ligand or agonist binding to TLR2, TLR3, TLR4, TLR5, TLR7, TLR8, TLR9 or TLR11.

12. The composition for enhancing immunogenicity according to claim 10 or 11, wherein the ligand or agonist binding to a Toll-like receptor (TLR) is any of the following (i) to (vii):
(i) a ligand or agonist binding to TLR2 selected from the group consisting of peptidoglycan, lipoprotein, lipopolysaccharide and zymosan;
(ii) a ligand or agonist binding to TLR3 selected from the group consisting of poly(I:C) and poly(A:U);
(iii) a ligand or agonist binding to TLR4 selected from the group consisting of lipopolysaccharide (LPS), HSP60, RS09, and MPLA;
(iv) flagellin as a ligand or agonist binding to TLR5;
(v) a ligand or agonist binding to TLR7 or 8 selected from the group consisting of an imidazoquinoline compound and single-strand RNA;
(vi) a ligand or agonist binding to TLR9 selected from the group consisting of bacterial DNA, unmethylated CpG DNA, hemozorin, ODN1585, ODN1668, and ODN1826; and
(vii) a ligand or agonist binding to TLR11 selected from the group consisting of profilin and uropathogenic bacteria.

13. The composition for enhancing immunogenicity according to any one of claims 1 to 12, wherein the number of molecules of the immuno-stimulating factor binding to one molecule of the modified amphiphilic polymer is 1 to 100.

14. The composition for enhancing immunogenicity according to any one of claims 1 to 13, wherein an average particle size of the particle is 0.1 to 50 µm.

15. A medicine containing the composition for enhancing immunogenicity according to any one of claims 1 to 14, as an active ingredient.

16. A vaccine containing the composition for enhancing immunogenicity according to any one of claims 1 to 14, as an active ingredient.

17. A vaccine for treatment and/or prevention of cancer, containing the composition for enhancing immunogenicity according to any one of claims 1 to 14, as an active ingredient.

18. A method for enhancing immunogenicity, comprising administering the composition for enhancing immunogenicity according to any one of claims 1 to 14, to a subject.

19. A method for treating and/or preventing cancer, comprising administering the composition for enhancing immunogenicity according to any one of claims 1 to 14, the medicine according to claim 15, or the vaccine according to claim 16 or 17, to a subject.
